(19) Europäisches Patentamt
European Patent Office
Office européen des brevets

(11) Numéro de publication : **0 031 784**
**B1**

(12) **FASCICULE DE BREVET EUROPÉEN**

(45) Date de publication du fascicule du brevet : **13.07.83**

(51) Int. Cl.³ : **C 07 C 67/293**, C 07 C 63/14

(21) Numéro de dépôt : **80420139.0**

(22) Date de dépôt : **09.12.80**

(54) **Procédé de préparation de carboxylates d'éthyle à partir de leurs homologues inférieurs.**

(30) Priorité : **21.12.79 FR 7932137**

(43) Date de publication de la demande :
**08.07.81 Bulletin 81/27**

(45) Mention de la délivrance du brevet :
**13.07.83 Bulletin 83/28**

(84) Etats contractants désignés :
**AT BE CH DE FR GB IT LI LU NL SE**

(56) Documents cités :
**EP A 0 004 732**
**EP A 0 031 606**
**FR A 2 359 811**
**FR A 2 397 389**
**US A 3 285 948**

(73) Titulaire : **RHONE-POULENC CHIMIE DE BASE**
**25, quai Paul Doumer**
**F-92408 Courbevoie (FR)**

(72) Inventeur : **Gauthier-Lafaye, Jean**
**21, rue Duguesclin**
**F-69006 Lyon (FR)**
Inventeur : **Perron, Robert**
**La Pecolière**
**F-69390 Charly (FR)**

(74) Mandataire : **Varniere-Grange, Monique et al**
**RHONE-POULENC RECHERCHES Centre de Recherches de Saint-Fons Service Brevets B.P. 62**
**F-69190 Saint-Fons (FR)**

**EP 0 031 784 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé de préparation de carboxylates d'éthyle à partir de leurs homologues inférieurs

La présente invention a pour objet un procédé de préparation de carboxylates d'éthyle, en particulier d'acétate d'éthyle, par réaction sur leurs homologues inférieurs, c'est-à-dire sur les carboxylates de méthyle correspondants, d'un mélange gazeux comprenant du monoxyde de carbone et de l'hydrogène.

Le procédé selon la présente invention peut être représenté par la réaction suivante :

$$R—CO—O—CH_3 + CO + 2H_2 \longrightarrow R—CO—O—CH_2—CH_3 + H_2O \qquad (1)$$

dans laquelle R représente un radical alkyle linéaire ou ramifié ayant de 1 à 16 atomes de carbone, un radical cycloalkyle ayant de 3 à 6 atomes de carbone, un radical cycloalkyle ayant de 3 à 6 atomes de carbone, un radical phényle ($C_6H_5$—), un radical $C_6H_5—C_xH_{2x}$— ou un radical $C_xH_{2x+1}—C_6H_4$—, x étant un entier compris entre 1 et 6 inclus.

R est avantageusement un radical alkyle ayant de 1 à 4 atomes de carbone tel que méthyle, éthyle, n-propyle, isopropyle, n-butyle, butyle secondaire, isobutyle et tertiobutyle.

Le procédé selon la présente invention convient plus particulièrement à la préparation de l'acétate d'éthyle à partir de l'acétate de méthyle.

Des auteurs (Cf. Journal of the American Chemical Society, 100 : 19, 1978, p. 6238-6239) ont montré qu'il est possible de préparer, en particulier, l'acétate d'éthyle par hydrocarbonylation de l'acétate de méthyle en présence simultanée de ruthénium, d'un promoteur iodé, d'un donneur de proton (qui est soit HI engagé initialement à la réaction ou formé in situ à partir de $CH_3I$, soit un acide carboxylique).

Toutefois, le développement à l'échelle industrielle d'une telle technique, dont l'intérêt de principe n'est pas contesté, est largement compromis par la faible activité du système catalytique mis en œuvre.

Récemment il a été proposé (Cf. demande de brevet français n° 78/20 843) d'effectuer cette réaction en présence d'un sel de cobalt et d'iode. Cependant les hautes pressions nécessaires pour que le système catalytique développe une activité acceptable ne laissent guère entrevoir de possibilité de développement industriel d'un tel procédé.

De cette analyse il ressort clairement qu'il serait souhaitable de pouvoir disposer d'un procédé pour la préparation de carboxylates d'alkyles à partir de leurs homologues inférieurs permettant d'opérer sous basse pression, avec une vitesse de réaction industriellement satisfaisante, sans pour autant perdre au niveau de la sélectivité en ester recherché.

La demanderesse a maintenant découvert un procédé permettant d'atteindre ces objectifs, souvent contradictoires par ailleurs.

La présente invention a donc pour objet un procédé de préparation de carboxylates d'éthyle par réaction sur leurs homologues inférieurs d'un mélange gazeux renfermant du monoxyde de carbone et de l'hydrogène caractérisé en ce qu'on effectue la réaction en présence simultanée dans le milieu réactionnel d'une quantité efficace de ruthénium, de cobalt, le rapport atomique Co/Ru étant inférieur ou égal à 1, d'au moins un iodure d'alkyle et d'au moins un iodure ionique minéral ou organique dont le cation est choisi dans le groupe constitué par les cations des métaux alcalins, les cations des métaux alcalino-terreux et les cations ammonium ou phosphonium quaternaire, la quantité totale de promoteurs iodés présente dans le milieu réactionnel étant telle que le rapport atomique I/Ru soit supérieur ou égal à 5.

La demanderesse a en effet trouvé de manière tout à fait inattendue que l'addition d'une faible quantité de cobalt à un système catalytique renfermant du ruthénium permet d'augmenter considérablement l'activité dudit système, même dans une gamme de pression à l'intérieur de laquelle le cobalt engagé isolément ne présente pratiquement pas d'activité.

Ainsi il apparaît que l'un des constituants essentiels du système catalytique selon la présente invention est le ruthénium. La forme précise sous laquelle le ruthénium est engagé à la réaction n'est pas fondamentale. Conviennent particulièrement bien à la mise en œuvre du présent procédé des ruthénium carbonyles tels que

$$Ru_3(CO)_{12},\ [Ru(CO)_3Br_2]_2,\ \text{et}\ Ru(CO)_4I_2$$

et plus généralement tout composé du ruthénium susceptible de conduire dans les conditions réactionnelles, à l'apparition *in situ* de ruthénium carbonyles. A ce titre on peut citer notamment le ruthénium métallique sous forme finement divisée, le tribromure de ruthénium, le triiodure de ruthénium, les carboxylates de ruthénium (en particulier l'acétate de ruthénium) et l'acétylacétonate de ruthénium.

La quantité de ruthénium à mettre en œuvre n'est pas critique. La teneur en ruthénium du milieu réactionnel ayant une influence positive sur la vitesse de réaction, sera déterminée en fonction de la vitesse que l'on estimera convenable d'atteindre.

De manière générale une quantité comprise entre 0,5 et 100 milliatomes-grammes de ruthénium par litre de milieu réactionnel (mAt-g/l) conduit à des résultats satisfaisants. On opère de préférence avec une teneur en ruthénium comprise entre 1 et 50 mAt-g/l de ruthénium.

Le second constituant essentiel du système catalytique est le cobalt. N'importe quelle source de

cobalt susceptible de réagir avec l'oxyde de carbone dans le milieu de réaction pour donner des complexes carbonyles du cobalt peut être utilisée dans le cadre du présent procédé.

Les sources typiques de cobalt sont par exemple le cobalt métallique finement divisé, des sels inorganiques tel que le nitrate ou le carbonate de cobalt, des sels organiques en particulier des carboxylates. Peuvent également être employés les cobalt-carbonyles ou hydrocarbonyles.

Parmi les dérivés du cobalt convenant pour la mise en œuvre du procédé selon l'invention, on peut citer le formiate, l'acétate, les halogénures de cobalt, et plus particulièrement l'iodure de cobalt et le dicobaltoctacarbonyle.

Une caractéristique du présent procédé réside dans le fait que la quantité de cobalt engagée à la réaction est telle que le rapport atomique Co/Ru soit toujours inférieur ou égal à 1. En général le rapport est compris entre 0,01 et 1 et de préférence entre 0,02 et 0,50 et de manière avantageuse entre 0,05 et 0,25.

Le procédé selon la présente invention exige également la présence dans le milieu réactionnel d'au moins un iodure d'alkyle, que l'on peut représenter par la formule R'—I, dans laquelle R' représente un radical alkyle linéaire ou ramifié ayant de 1 à 5 atomes de carbone, ou un radical $C_6H_5$—$C_xH_{2x}$—, x ayant la signification précédente, R et R' pouvant par ailleurs être identiques.

Bien entendu l'iodure d'alkyle qui peut être chargé initialement, est susceptible d'être formé *in situ* dans les conditions réactionnelles notamment à partir d'iode, d'acide iodhydrique, d'un iodure d'acyle (R'—CO—I), d'iodure de cobalt et/ou d'iodure de ruthénium. En d'autres termes une partie ou la totalité de l'iodure d'alkyle dont la présence dans le milieu réactionnel est nécessaire pour la mise en œuvre du présent procédé peut être formée à partir de ses précurseurs définis ci-avant.

On constatera en outre que lorsque le dérivé iodé est choisi parmi les composés du cobalt ou du ruthénium, il peut être considéré comme précurseur non seulement de l'iodure d'alkyle mais encore comme précurseur du (ou des) catalyseur(s) métallique(s).

Dans le cadre de la présente invention, les iodures d'alkyles inférieurs ayant de 1 à 4 atomes de carbone, constituent une classe préférée d'iodures d'alkyles. Les iodures de méthyle et d'éthyle conviennent particulièrement bien à la mise en œuvre du procédé selon l'invention.

Pour une bonne mise en œuvre du présent procédé une concentration en iodure d'alkyle d'au moins 5 millimoles par litre de milieu réactionnel (mMol/l) est généralement requise. Toutefois il n'y a pas d'avantage à dépasser une concentration de 500 mMol/l, les risques de corrosion de l'appareillage étant susceptible d'augmenter du fait que, comme le montre la réaction (1) ci-avant, il y a formation d'eau.

Le procédé selon la présente invention exige en outre la présence dans le milieu réactionnel d'au moins un iodure ionique minéral ou organique, dont le cation est choisi dans le groupe constitué par les cations des métaux alcalins, les cations des métaux alcalino-terreux et les cations ammonium ou phosphonium quaternaire représentés par les formules I à III ci-après :

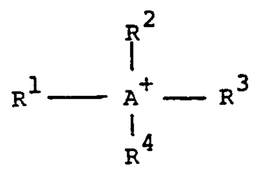
(I)

dans laquelle A représente un atome d'azote ou de phosphore, $R_1$, $R_2$, $R_3$ et $R_4$, pouvant être identiques ou différents représentent l'hydrogène ou, de préférence, des radicaux organiques dont la valence libre est portée par un atome de carbone, deux quelconques de ces divers radicaux pouvant éventuellement former ensemble un radical unique divalent.

Plus spécifiquement $R_1$, $R_2$, $R_3$ et $R_4$ peuvent représenter des radicaux alkyles linaires ou ramifiés, des radicaux cycloalkyles, aralkyles (par exemple benzyle) ou aryles monocycliques, ayant au plus 16 atomes de carbone, pouvant le cas échéant être substitués par 1 à 3 radicaux alkyles ayant de 1 à 4 atomes de carbone ; deux des radicaux $R_1$ à $R_4$ pouvant éventuellement former ensemble un radical unique divalent — alkylène ou alcénylène — comportant 3 à 6 atomes de carbone (par exemple un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone.

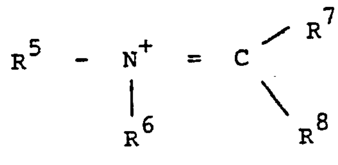
(II)

dans laquelle $R_5$, $R_6$, $R_7$ et $R_8$ identiques ou différents représentent des radicaux alkyles ayant de 1 à 4 atomes de carbone, l'un des radicaux $R_7$ ou $R_8$ pouvant en outre représenter l'hydrogène, $R_7$ et $R_8$ pouvant éventuellement former ensemble un radical unique divalent alkylène comportant de 3 à 6 atomes de carbone, tétraméthylène ou hexaméthylène par exemple ; $R_6$ et $R_7$ ou $R_8$ peuvent former ensemble un radical unique divalent — alkylène ou alcénylène — comportant 4 atomes de carbone et le cas échéant 1 ou 2 doubles liaisons éthyléniques, l'atome d'azote étant alors inclus dans un hétérocycle, pour constituer, par exemple, un cation pyridinium.

$$(R_9)_2\underset{\underset{R^5}{|}}{A^+} - (CH_2)_y - \underset{\underset{R^5}{|}}{A^+}(R^9)_2 \qquad \text{(III)}$$

dans laquelle $R_5$ et $A^+$ ont la signification donnée précédemment, $R_9$ pouvant être identique à $R_5$ représente un radical alkyle ayant de 1 à 4 atomes de carbone ou un radical phényle, et y est un entier compris entre 1 et 10 inclus et de préférence entre 1 et 6 inclus. À titre d'exemple d'iodures d'ammonium quaternaire convenant à la mise en œuvre du présent procédé on peut citer les iodures

de tétraméthylammonium
de triéthylméthylammonium
de tributylméthylammonium,
de triméthyl(n-propyl)ammonium,
de tétraéthylammonium,
de tétrabutylammonium,
de dodécyltriméthylammonium,
de benzyltriméthylammonium,
de benzyldiméthylpropylammonium,
de benzyldiméthyloctylammonium,
de diméthyldiphénylammonium,
de méthyltriphénylammonium,
de N,N-diméthyl-triméthylènammonium,
de N,N-diéthyl-triméthylènammonium,
de N,N-diméthyl-tétraméthylènammonium,
de N,N-diéthyl-tétraméthylènammonium,
de N-méthylpyridinium
de N-éthylpyridinium
de N-méthylpicolinium

A titre d'exemple d'iodures de phosphonium quaternaire convenant également à la mise en œuvre du présent procédé, on peut citer les iodures

de tétraméthylphosphonium,
d'éthyltriméthylphosphonium,
de triméthylpentylphosphonium,
d'octyltriméthylphosphonium,
de dodécyltriméthylphosphonium,
de triméthylphénylphosphonium,
de diéthyldiméthylphosphonium,
de dicyclohexyldiméthylphosphonium,
de diméthyldiphénylphosphonium,
de cyclohexyltriméthylphosphonium,
de triéthylméthylphosphonium,
de méthyl-tri(isopropyl)phosphonium,
de méthyl-tri(n-propyl)phosphonium,
de méthyl-tri(n-butyl)phosphonium,
de méthyl-tris(méthyl-2 propyl)phosphonium,
de méthyltricyclohexylphosphonium,
de méthyltriphénylphosphonium,
de méthyltribenzylphosphonium,
de méthyl-tris(méthyl-4 phényl)phosphonium,
de méthyltrixylylphosphonium,
de diéthylméthylphénylphosphonium,
de dibenzylméthylphénylphosphonium,
d'éthyltriphénylphosphonium,
de tétraéthylphosphonium,
d'éthyl-tri(n-propyl)phosphonium,
de triéthylpentylphosphonium,
d'éthyltriphénylphosphonium,
de n-butyl-tri(n-propyl)phosphonium,
de butyltriphénylphosphonium,
de benzyltriphénylphosphonium,
de (β-phényléthyl)diméthylphénylphosphonium,
de tétraphénylphosphonium,
de triphényl(méthyl-4 phényl)phosphonium

La nature précise du cation ammonium ou phosphonium quaternaire n'est pas fondamentale dans le cadre du présent procédé. Le choix parmi ces composés est davantage orienté par des considérations d'ordre pratique, telles que la solubilité dans le milieu réactionnel, la disponibilité et la commodité d'emploi.

A ce titre, les iodures d'ammonium ou de phosphonium quaternaire représentés soit par la formule (I) dans laquelle l'un quelconque des radicaux $R_1$ à $R_4$ est choisi parmi les radicaux alkyles linéaires ayant de 1 à 4 atomes de carbone, soit par les formules (II) ou (III) dans laquelle $R_5$ (ou $R_6$) est également un radical alkyle ayant de 1 à 4 atomes de carbone, conviennent particulièrement bien.

En outre, parmi les iodures d'ammonium on préfère ceux dont les cations correspondent à la formule (I) dans laquelle tous les radicaux $R_1$ à $R_4$ sont choisis parmi les radicaux alkyles linéaires ayant de 1 à 4 atomes de carbone et dont au moins trois d'entre eux sont identiques.

De même, parmi les iodures de phosphonium quaternaire, on préfère ceux dont les cations correspondent à la formule (I) dans laquelle l'un quelconque des radicaux $R_1$ à $R_4$ représente un radical alkyle linéaire ayant de 1 à 4 atomes de carbone, les trois autres radicaux étant identiques et choisis parmi les radicaux phényle, tolyle ou xylyle.

Les iodures des métaux alcalins, en particulier les iodures de lithium, de potassium et de sodium constituent une classe préférée d'iodures ioniques dans le cadre de la présente invention. Les iodures de phosphonium quaternaire et plus particulièrement ceux dont les cations correspondent à la formule (I) ci-avant, dans laquelle l'un des radicaux $R_1$ à $R_4$ est un radical alkyle ayant de 1 à 4 atomes de carbone, les trois autres radicaux étant identiques et choisis parmi les radicaux phényle, tolyle ou xylyle, constituent une autre classe d'iodures ioniques particulièrement efficaces pour la mise en œuvre de la présente invention.

Si la quantité d'iodure ionique qui doit être présente dans le milieu réactionnel peut varier dans de larges limites, elle est en général telle que le rapport $I^-/Ru$ soit compris entre 1 et 100 ; ce rapport est avantageusement fixé à une valeur située dans la gamme allant de 2 à 50.

Selon une caractéristique essentielle du présent procédé, la quantité totale de promoteurs iodés présente dans le milieu réactionnel est telle que le rapport atomique I/Ru soit supérieur ou égal à 5 ; il n'est pas utile de dépasser la valeur de 100. De manière avantageuse, la quantité totale de promoteurs iodés présente dans le milieu réactionnel est telle que le rapport atomique I/Ru soit compris entre 10 et 50.

La Demanderesse a constaté de manière surprenante que l'adjonction d'un cocatalyseur alcalin ou alcalinoterreux au milieu réactionnel renfermant du cobalt, du ruthénium, le rapport atomique Co/Ru étant inférieur ou égal à 1, un iodure d'alkyle et un iodure ionique et défini ci-avant, a une influence notable sur la sélectivité en ester recherché. A cet effet on peut utiliser des sels minéraux ou organiques du sodium, du potassium, du lithium, du césium, du rubidium, du calcium ou du magnésium et plus particulièrement, les oxydes, les hydroxydes, les carbonates, les nitrates, les alcoolates ($R''—O^-$) et les carboxylates ($R''—CO—O^-$) des métaux précités, R″ ayant la signification donnée précédemment pour R, R″ et R pouvant être identiques ou différents. Des carboxylates et plus particulièrement les acétates sont d'un emploi commode et à ce titre peuvent être préconisés. Les acétates de lithium, de sodium, de potassium et de magnésium conviennent particulièrement bien à la mise en œuvre du présent procédé.

Bien que l'utilisation de ce cocatalyseur constitue un aspect avantageux du présent procédé, elle n'est en aucun cas obligatoire.

Une quantité de cocatalyseur ou l'ordre de 1 à 500 atomes-grammes de métal alcalin ou alcalino-terreux par atome-gramme de ruthénium présent dans le milieu réactionnel conduit à des résultats satisfaisants, bien que des quantités inférieures ou supérieures puissent être utilisées. De bons résultats sont obtenus lorsque le rapport atomique du métal alcalin ou alcalino-terreux au ruthénium est compris entre 2 et 250.

De bons résultats sont obtenus, en particulier lorsqu'on charge initialement de l'iodure de méthyle, un iodure alcalin et un sel de magnésium ou bien lorsqu'on charge initialement de l'iodure de méthyle, un iodure de phosphonium quaternaire et un sel de lithium.

La Demanderesse a également constaté que lorsqu'on charge initialement un iodure d'alkyle et/ou l'un de ses précurseurs définis précédemment en quantité telle que le rapport I/Ru soit supérieur ou égal à 5, on peut remplacer dans la charge initiale les iodures alcalins ou alcalinoterreux par l'un quelconque des sels métalliques correspondants, cités ci-avant. Dans le cadre de cette variante d'exécution la quantité de sels chargée est telle que le rapport molaire du métal alcalin ou alcalino-terreux à l'iodure d'alkyle soit compris entre 0,1 et 10 inclus. Ce rapport est avantageusement fixé à une valeur comprise entre 0,25 et 5, inclus. De bons résultats sont obtenus lorsqu'on charge initialement conjointement de l'iodure de méthyle en quantité telle que le rapport $CH_3I/Ru$ soit supérieur ou égal à 5 et un acétate de lithium, de sodium, de potassium ou de magnésium.

Le procédé selon la présente invention est conduit de préférence en phase liquide. Il est possible d'utiliser des solvants ou diluants, en particulier un acide carboxylique de formule $R''—COOH$, dans laquelle R″ à la signification donnée précédemment pour R, R″ et R pouvant être identiques ou différents, l'acide carboxylique ne correspondant pas nécessairement aux carboxylates alcalin (ou alcalino-terreux) dont il a été question ci-avant. Toutefois, la présence d'un tel solvant ne constitue qu'une variante avantageuse du présent procédé.

On remarquera qu'il y a production d'eau dans la réaction (1) décrite ci-avant. La Demanderesse a

constaté en outre que la présence d'eau n'est pas nuisible à la bonne marche du procédé et qu'au contraire, dans certains cas, la présence d'eau initialement chargée tend à favoriser la réaction. Aussi on peut utiliser les réactifs de qualité technique renfermant le cas échéant, jusqu'à 10 % en poids d'eau environ.

Conformément à la présente invention, le système catalytique et la matière de départ, définis ci-avant, ainsi que, le cas échéant, des solvants ou diluants, sont introduits dans un réacteur résistant à la pression et conçu dans un matériau approprié, sous atmosphère d'hydrogène et de monoxyde de carbone. Le rapport molaire $H_2/CO$ qui est donné par la stœchiométrie du schéma réactionnel en tête du présent mémoire, peut néanmoins varier dans de larges limites et est généralement compris entre 1/5 et 5/1, et de manière avantageuse entre 1/1 et 3/1. Bien entendu, le mélange de gaz peut renfermer des impuretés, en particulier du dioxyde de carbone, de l'oxygène, du méthane et/ou de l'azote.

Le réacteur est alors porté à la température réactionnelle. Une température généralement supérieure à 150 °C doit être préconisée. Cette température est plus particulièrement comprise entre 175 et 250 °C ; de bons résultats sont obtenus dans la gamme de températures comprises entre 190 °C et 230 °C.

On opère sous pression. La pression totale est généralement supérieure à 50 bars, et peut être aussi élevée que 600 bars. Néanmoins le nouveau système catalytique, objet de la présente invention, permet d'obtenir des résultats appréciables dans une gamme de pressions comprises entre 150 et 350 bars.

Un avantage supplémentaire du présent procédé réside dans le fait que la faible proportion de sous-produits liquides formés est constituée essentiellement de produits recyclables.

En fin de réaction, les produits obtenus peuvent être aisément séparés, par distillation fractionnée du mélange résultant, par exemple ; les sous-produits, notamment l'acide carboxylique formé, peuvent être recyclés à la réaction.

Les exemples ci-après illustrent la présente invention, sans toutefois en limiter le domaine ou l'esprit.

Dans ce qui suit on a utilisé les conventions suivantes :

AcOEt désigne l'acétate d'éthyle
MeOH désigne le méthanol
AcH désigne l'acétaldéhyde
EtOH désigne l'éthanol
AcOH désigne l'acide acétique
V : nombre de moles de monoxyde de carbone absorbées par seconde et par mole de cobalt engagé à la réaction.

## Exemple 1

Dans un autoclave en acier inoxydable Z-8 CNDT 17-12 (norme AFNOR) de 250 ml de capacité, on charge :

80 ml d'acétate de méthyle (1 002 m.Mol)
20 ml d'acide acétique (350 m.Mol)
510 mg d'iodure de méthyle (3,54 m.Mol)
4,85 g d'iodure de méthyltriphénylphosphonium (12 m.Mol)
36,8 mg de dicobaltoctacarbonyle (0,22 mAt-g de Co)
279,2 mg de triruthéniumdodécacarbonyle (1,31 mAt-g de Ru)
50 m Mol d'acétate de lithium

Après fermeture de l'autoclave on établit une pression de 140 bars à l'aide d'un mélange $H_2/CO$ = 2/l (molaire). L'agitation par un système de va-et-vient est mise en route et l'autoclave est porté à 215 °C, en 25 minutes environ, au moyen d'un four annulaire. La pression dans l'autoclave est alors de 220 bars et elle est maintenue entre 230 et 260 bars par des recharges successives du mélange $H_2/CO$ initial.

Après 40 minutes de réaction à la température indiquée, le chauffage et l'agitation sont arrêtés ; l'autoclave est refroidi et dégazé. Le mélange réactionnel résultant est analysé par chromatographie gazeuse (après dilution dans un mélange 56/44 d'eau et de diméthoxy-1,2 éthane et acidification à l'acide sulfurique 36 N). Il contient 24,25 g d'acétate d'éthyle à côté de 1,85 g de méthanol, 0,45 g d'acétaldéhyde, 1,95 g d'éthanol et 35,45 g d'acide acétique.

Les productivités de la réaction en acétate d'éthyle sont donc de :

365 grammes par heure et par litre (g/h × 1),
270 grammes par heure et par gramme de ruthénium (g/h×gRu),
2 900 grammes par heure et par gramme de cobalt (g/h × gCo).

## Essais témoins (a) à (e)

On a réalisé une série d'essais selon le mode opératoire décrit pour l'exemple 1 ci-avant. Ces essais n'entrent pas dans le cadre du présent procédé.

Dans l'essai témoin (a) on a omis de charger le dicobaltoctacarbonyle.

Dans l'essai témoin (b) on a omis de charger le triruthéniumdodécarbonyle.

Dans l'essai témoin (c) on a remplacé le dicobaltoctacarbonyle par une quantité équivalente de ferpentacarbonyle.

(0,21 mAt-g de Fer).

Dans l'essai témoin (d) on a remplacé le triruthéniumdodécacarbonyle par une quantité équivalente de ferpentacarbonyle.

(1,31 mAt-g de Fer).

Dans l'essai témoin (e) on a omis de charger l'iodure de méthyltriphénylphosphonium, de ce fait le rapport I/Ru n'est que de 2,7.

Les conditions particulières ainsi que les résultats obtenus figurent dans le tableau I ci-après dans lequel on a rappelé certaines conditions et les résultats obtenus dans l'exemple 1.

Exemples 2 à 4

Dans l'autoclave et selon le mode opératoire décrit ci-avant on réalise une série d'essais en chargeant lors de chaque essai :

— 0,215 mAt-g de cobalt sous la forme de dicobaltoctacarbonyle
— 1,31 mAt-g de ruthénium sous la forme de triruthéniumdodécacarbonyle.
— 12 mMol d'iodure de sodium (exemple 3 et 4) ou 12 mMol d'iodure de méthyltriphénylphosphonium (exemple 2).

Les conditions opératoires communes sont les suivantes :

— $H_2/CO$ : 2/l (molaire)
— Température : 215 °C
— Pression totale à la température indiquée : 260 bars
— Durée de l'essai à la température indiquée : 1 heure 15 minutes.

Les conditions particulières ainsi que les résultats obtenus sont portés dans le tableau (II) ci-après.

Exemples 5 à 11

Dans l'autoclave et selon le mode opératoire décrit ci-avant on réalise une série d'essais en chargeant lors de chaque essai :

— 1 000 mMol d'acétate de méthyle
— 350 mMol d'acide acétique
— 12 mMol d'iodure de sodium
— 1,31 mAt-g de ruthénium sous forme de triruthéniumdodécacarbonyle
— de l'iodure de méthyle et du dicobaltoctacarbonyle, sauf mention contraire.

Les conditions opératoires communes sont les suivantes :

— $H_2/CO$ : 2/l (molaire)
— Température : 215 °C
— Pression totale à la température indiquée : 260 bars

(sauf indications contraires).

Les conditions particulières ainsi que les résultats obtenus figurent dans le tableau (III) ci-après, dans lequel Mg $(OAC)_2$ désigne l'acétate de magnésium tétrahydraté.

N.B. : La quantité d'eau indiquée dans le tableau (III), ne comprend pas l'eau d'hydratation éventuellement introduite avec le sel alcalin.

Exemple 12 à 16

Dans l'autoclave et selon le mode opératoire décrit ci-avant on réalise une série d'essais en chargeant lors de chaque essai :

— 0,22 mAt-g de cobalt sous la forme de dicobaltoctacarbonyle
— du ruthénium sous forme de triruthéniumdodécacarbonyle
— 3,55 mMol d'iodure de méthyle
— 12 mMol d'iodure de méthyltriphénylphosphonium

— 1 000 mMol d'acétate de méthyle
— 350 mMol d'acide acétique

(sauf indications contraire).

Les conditions opératoires communes sont les suivantes :

— $H_2/CO$ : 2/l (molaire)
— Température : 215 °C
— Pression totale à la température indiquée : 260 bars
— Durée de l'essai à la température indiquée : en principe 1 heure 15 minutes.

Les conditions particulières ainsi que les résultats obtenus figurent dans le tableau IV ci-après.
N.B. : Dans les tableaux (III) et (IV) S désigne le rapport molaire :

$$\frac{\text{(Ac Œt) formé}}{\text{(Ac Œt + Et OH + Ac OH + Ac H) formés}} \times 100$$

Exemples 17 à 20

Dans l'autoclave et selon le mode opératoire décrit précédemment, on réalise une série d'essais sur une charge constituée de :

— 75 ml d'acétate de méthyle
— 20 ml d'acide acétique
— 5 ml d'eau
— 18 mMol d'iodure de méthyle
— 0,22 mMol d'acétate de cobalt tétrahydraté
— 1,31 mAt-g de ruthénium sous forme de triruthéniumdodécacarbonyle
— de l'acétate de sodium dont la quantité chargée figure dans le tableau (V) ci-après.

Les conditions opératoires communes sont les suivantes :

— $H_2/CO$ : 2/l (molaire)
— Température : 215 °C
— Pression totale à la température indiquée : 260 bars
— Durée de l'essai à la température indiquée : 40 minutes.

Les conditions particulières ainsi que les résultats obtenus figurent dans le tableau (V) ci-après.
L'essai témoin f est réalisé en l'absence d'acétate de sodium.

Exemples 21 à 23

Dans l'autoclave et selon le mode opératoire décrit précédemment, on réalise une série d'essais sur une charge constituée de :

— 75 ml d'acétate de méthyle
— 20 ml d'acide acétique
— 5 ml d'eau
— 18 mMol d'iodure de méthyle
— 0,22 mMol d'acétate de cobalt tétrahydraté
— 1,31 mAt-g de ruthénium sous forme de triruthéniumdodécacarbonyle
— de l'acétate de lithium dont la quantité chargée figure dans le tableau (VI) ci-après.

Les conditions opératoires communes sont les suivantes :

— $H_2/CO$ : 2/l (molaire)
— Température : 215 °C
— Pression totale à la température indiquée : 260 bars
— Durée de l'essai à la température indiquée : 40 minutes.

Les conditions particulières ainsi que les résultats obtenus figurent dans le tableau (VI) ci-après.
L'essai témoin f est réalisé en l'absence d'acétate de lithium.

Exemple 24

Dans l'appareillage et selon le mode opératoire décrit précédemment on réalise un essai sur une charge constituée de 77 ml d'acétate de méthyle, 20 ml d'acide acétique, 3 ml d'eau, 35 mMol d'iodure de méthyle, 0,22 mMol d'acétate de cobalt hydraté, 1,31 m At-g de ruthénium sous forme de triruthénium dodécarbonyle et 17 mMol d'acétate de lithium. Après 40 mn de réaction à 215 °C sous une pression totale maintenue à 250 bars par des recharges d'un mélange $H_2/CO$ = 2/l (molaire) on dose :

Ac Œt = 7,37 g (Pr = 220 g/h × l)
Me OH = 2,92 g
Ac H = 0,90 g
Et OH = 1,79 g
Ac OH = 34,5 g

Exemple 25

On reproduit l'exemple 24 ci-avant en ne modifiant que le volume d'acétate de méthyle chargé (80 ml) et la quantité d'iodure de méthyle (30 mMol)
Les résultats obtenus sont les suivants :

Ac Œt = 8,71 g (Pr = 260 g/h × l)
Me OH = 1,88 g
Ac H = 0,58 g
Et OH = 1,80 g
Ac OH = 33,2 g

Exemple 26

On reproduit l'exemple 25 ci-avant en remplaçant l'acétate de lithium par 30 mMol d'acétate de sodium.
Les résultats obtenus sont les suivants :

Ac Œt = 11,7 g (Pr = 350 g/h × l)
Me OH = 2,57 g
Ac H = 1,02 g
Et OH = 1,62 g
Ac OH = 35,6 g

Exemple 27

On reproduit l'exemple 25 en remplaçant l'acétate de lithium par 17 mMol d'acétate de magnésium tétrahydraté.
Les résultats sont les suivants :

Ac Œt = 8,50 g (Pr = 260 g/h × l)
Me OH = 1,72 g
Ac H = 1,18 g
Et OH = 1,05 g
Ac OH = 35,1 g

Exemple 28

Dans l'autoclave et selon le mode opératoire décrit précédemment on fait réagir un mélange d'hydrogène et de monoxyde de carbone dans le rapport (molaire) 2/l, sur une charge constituée de :

— 80 ml d'acétate de méthyle
— 20 ml d'acide acétique
— 3 ml d'eau
— 0,22 m Mol d'iodure de cobalt
— 1,31 m At-g de ruthénium sous forme de triruthéniumdodécacarbonyle
— 30 m Mol d'iodure de sodium.

Après 20 minutes de réaction à 215 °C, la pression totale étant alors de 250 bars, on dose :

Ac Œt = 6,88 g (Pr = 210 g/h × l)
Me OH = 2,41 g
Ac H = 0,49 g
Et OH = 2,08 g
Ac OH = 35,3 g

Exemple 29

Dans l'autoclave et selon le mode opératoire décrit précédemment on fait réagir un mélange d'hydrogène et de monoxyde de carbone dans le rapport (molaire) 2/l, sur une charge constituée de :

— 76 ml d'acétate de méthyle
— 20 ml d'acide acétique
— 3 ml d'eau
— 0,22 m Mol d'acétate de cobalt tétrahydraté
— 1,31 m At-g de ruthénium sous forme de triruthéniumdodécacarbonyle
— 15 m Mol d'iodure de lithium
— 15 m Mol d'iodure de méthyltriphénylphosphonium

Après 20 minutes de réaction à 215 °C, la pression totale étant alors de 250 bars, on dose :

Ac Œt = 7,79 g (Pr = 230 g/h × l)
Me OH = 1,97 g
Ac H = 2,77 g
Et OH = 1,09 g
Ac OH = 39,6 g
$CH_3$ I = 650 mg

Exemple 30

Dans l'autoclave et selon le mode opératoire décrit précédemment on fait réagir un mélange d'hydrogène et de monoxyde de carbone dans le rapport (molaire) 2/l, sur une charge constituée de :

— 80 ml d'acétate de méthyle
— 20 ml d'acide acétique
— 3 ml d'eau
— 0,22 m Mol d'acétate de cobalt tétrahydraté
— 1,31 m At-g de ruthénium sous forme de triruthéniumdodécacarbonyle
— 30 m Mol d'iodure de sodium
— 17 m Mol d'acétate de magnésium tétrahydraté

Après 20 minutes de réaction à 215 °C, la pression totale étant alors de 250 bars, on dose :

Ac Œt = 8,39 g (Pr = 250 g//h × l)
Me OH = 1,99 g
Ac H = 0,99 g
Et OH = 2,08 g
Ac OH = 37,4 g
$CH_3$ I = 370 mg

(voir tableaux page 11)

TABLEAU I

| REF. | CATALYSEUR | | DUREE DE REACTION | AcOET (g) | Me OH (g) | AcH (g) | Et OH (g) | Ac OH (g) | V |
|---|---|---|---|---|---|---|---|---|---|
| 1 | Ru | Co | 40 mn | 24,25 | 1,85 | 0,45 | 1,95 | 35,45 | 0,77 |
| a | Ru | - | 1 h 15 mn | 6,30 | 1,5 | - | - | 30,70 | ND |
| b | - | Co | 1 h 15 mn | 0,9 | 1,2 | 2,75 | - | 29,70 | 0,11 |
| c | Ru | Fe | 1 h 15 mn | 8,60 | 0,4 | - | - | 24,10 | ND |
| d | Fe | Co | 1 h 15 mn | 0 | - | 2,45 | - | 30,35 | 0,12 |
| e | Ru | Co | 1h 15 mn | 6,30 | 0,65 | - | 0,25 | 22,80 | 0,08 |

Ce tableau démontre clairement le caractère particulier de l'association d'une faible quantité de cobalt au ruthénium. D'autre part, l'essai témoin (e) met en évidence l'importance de la quantité de promoteurs iodés présente dans le milieu réactionnel.

ND = non déterminable.

TABLEAU II

| | CHARGE | | | | AcOEt | V |
|---|---|---|---|---|---|---|
| Ex. n° | AcOMe (m.Mol) | Ac OH (m.Mol) | $H_2O$ (m.Mol) | $CH_3I$ (m.Mol) | (g) | |
| 2 | 1253 | 0 | 0 | 3,54 | 9,3 | 0,37 |
| 3 | 1000 | 350 | " | " | 18,0 | 0,48 |
| 4 | " | 350 | 170 | 3,62 | 15,9 | 0,27 |

(voir tableau III page 12)

TABLEAU III

| Ex. n° | $H_2O$ m.Mol | $CH_3I$ m.Mol | Co m.At-g | COCATALYSEUR nature | COCATALYSEUR m.Mol | Durée de réaction | Ac OEt g | Ac OEt g/hxl | V | S |
|---|---|---|---|---|---|---|---|---|---|---|
| 5* | 170 | 3,66 | 0,22 | Li OAc | 50 | 2h 20 mn | 7,75 | 35 | 0,08 | 63 |
| 6 | 67 | ** | 1,08 | $Mg(OAc)_2$ | 25 | 40mn | 5,90 | 90 | 0,03 | 84 |
| 7 | " | 7,04 | 0,12 | " | " | " | 7,95 | 120 | 0,43 | 72 |
| 8 | " | 7,02 | 0,22 | " | " | " | 10,90 | 160 | 0,35 | 67 |
| 9 | " | 3,54 | " | " | " | " | 14,80 | 220 | 0,41 | 82 |
| 10 | 170 | 3,52 | " | Li OAc | 5 | 1h 15 mn | 20,65 | 165 | 0,30 | 81 |
| 11 | " | 3,62 | 0,21 | Li OAc | 50 | " | 17,95 | 145 | 0,32 | 68 |

* Exemple réalisé à 170 °C, avec un rapport molaire $H_2/CO$ : I, sous 230 bars

** Exemple réalisé avec 474 mMol d'$I_2$, 1.08 m.At-g de Co sous forme de $CoI_2$, et sous 160 bars.

TABLEAU IV

| Nº Ex. | Ru m.At-g | COCATALYSEUR Nature | m. Mol | Ac OEt g | g/h x 1 | V | S |
|---|---|---|---|---|---|---|---|
| 12(*) | 1,31 | Li OAc | 50 | 21,8 | 175 | 0,51 | 51 |
| 13(**) | 1,31 | " | 5 | 15,35 | 155 | 0,52 | 44 |
| 14(**) | 1,31 | " | 15 | 21,35 | 210 | 0,5 | 56 |
| 15 | 1,33 | KOAc | 50 | 18,75 | 150 | 0,27 | 83 |
| 16 | 0,22 | Li OAc | 50 | 5,25 | 40 | 0,21 | 29 |

* Exemple réalisé sur 1 253 m.Mol d'acétate de méthyle et sans introduire d'acide acétique dans la charge de départ.
** La durée de l'essai est de 1 heure.

TABLEAU V

| Ex. nº | Ac ONa m.Mol | Ac OEt (g) | Me OH (g) | Ac H (g) | Et OH (g) | Ac OH (g) |
|---|---|---|---|---|---|---|
| 17 | 10 | 4,93 | 2,56 | 1,14 | 1,17 | 33,9 |
| 18 | 20 | 9,84 | 2,39 | 0,34 | 1,72 | 32,5 |
| 19 | 35 | 8,27 | 2,03 | 0,06 | 1,26 | 32,7 |
| 20 | 50 | 5,90 | 1,58 | | 0,64 | 30,6 |
| f | 0 | 2,86 | 2,64 | | 0,60 | 30,4 |

TABLEAU VI

| Ex. nº | Ac OLi m. Mol | Ac OEt (g) | Me OH (g) | Ac H (g) | Et OH (g) | Ac OH (g) | Pr |
|---|---|---|---|---|---|---|---|
| 21 | 10 | 5,45 | 2,51 | 0,67 | 1,73 | 32,7 | 160 |
| 22 | 25 | 10,7 | 2,18 | 0,57 | 2,20 | 37,3 | 300 |
| 23 | 40 | 9,26 | 2,12 | 0,40 | 2,08 | 38,6 | 280 |
| f | 0 | 2,86 | 2,64 | - | 0,60 | 30,4 | 57 |

**Revendications**

1. Procédé de préparation de carboxylates d'éthyle par réaction sur les carboxylates de méthyle correspondants d'un mélange renfermant du monoxyde de carbone et de l'hydrogène en phase liquide, à une température comprise entre 175 et 250 °C sous une pression totale comprise entre 50 et 600 bars en présence de ruthénium et d'un promoteur iodé, le rapport atomique I/Ru étant supérieur ou égal à 5, caractérisé en ce qu'on opère simultanément en présence de cobalt, le rapport atomique Co/Ru étant inférieur ou égal à 1, d'au moins un iodure d'alkyle et d'au moins un iodure ionique minéral ou organique dont le cation est choisi dans le groupe constitué par les cations des métaux alcalins, les cations des métaux alcalino-terreux et les cations ammonium ou phosphonium quaternaire.

2. Procédé selon la revendication 1, caractérisé en ce que le carboxylate de méthyle a pour formule $R—CO—OCH_3$ dans laquelle R représente un radical alkyle linéaire ou ramifié ayant de 1 à 16 atomes de carbone, un radical cycloalkyle ayant de 3 à 6 atomes de carbone, un radical phényle ($C_6H_5—$), un radical $C_6H_5—C_xH_{2x}—$ ou un radical $C_xH_{2x+1}—C_6H_4—$, x étant un entier compris entre 1 et 6 inclus.

3. Procédé selon la revendication 2, caractérisé en ce que R représente un radical alkyle ayant au plus 4 atomes de carbone.

4. Procédé selon la revendication 1, caractérisé en ce que la quantité de ruthénium est comprise entre 0,5 et 100 mAt-g, et de préférence entre 1 et 50 mAt-g par litre de milieu réactionnel.

5. Procédé selon la revendication 1 ou 4, caractérisé en ce que le rapport atomique Co/Ru est compris entre 0,01 et 1, et de préférence, entre 0,02 et 0,50.

6. Procédé selon la revendication 1, caractérisé en ce que l'iodure d'alkyle utilisé est l'iodure de méthyle.

7. Procédé selon l'une quelconque des revendications précédentes, caractérisé en ce que le cation de l'iodure ionique est choisi parmi les cations des métaux alcalins.

8. Procédé selon l'une quelconque des revendications 1 à 6, caractérisé en ce que le cation de l'iodure ionique est choisi parmi les cations ammonium ou phosphonium quaternaire de formule I à III ci-après :

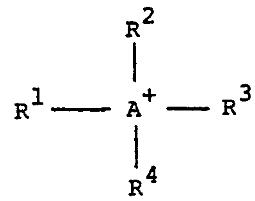

(I)

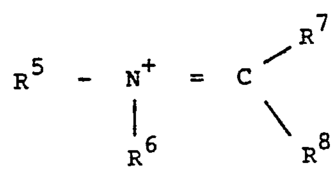

(II)

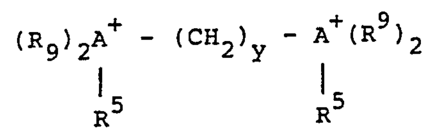

(III)

dans lesquelles

A représente un atome d'azote ou de phosphore,

$R_1$, $R_2$, $R_3$ et $R_4$, pouvant être identiques ou différents représentent l'hydrogène ou, de préférence, des radicaux organiques dont la valence libre est portée par un atome de carbone, deux quelconques de ces divers radicaux pouvant éventuellement former ensemble un radical unique divalent.

$R_5$, $R_6$, $R_7$ et $R_8$ identiques ou différents représentent des radicaux alkyles ayant de 1 à 4 atomes de carbone, l'un des radicaux $R_7$ ou $R_8$ pouvant en outre représenter l'hydrogène, $R_7$ et $R_8$ pouvant éventuellement former ensemble un radical unique divalent alkylène comportant de 3 à 6 atomes de carbone, tétraméthylène ou hexaméthylène par exemple ; $R_6$ et $R_7$ ou $R_8$ peuvent former ensemble un radical unique divalent — alkylène ou alcénylène — comportant 4 atomes de carbone et le cas échéant 1 ou 2 doubles liaisons éthyléniques, l'atome d'azote étant alors inclus dans un hétérocycle,

$R_9$ pouvant être identique à $R_5$ représente un radical alkyle ayant de 1 à 4 atomes de carbone ou un radical phényle, et y est un entier compris entre 1 et 10 inclus et de préférence entre 1 et 6 inclus.

9. Procédé selon la revendication 8, caractérisé en ce que le cation de l'iodure ionique est choisi parmi les cations de formule (I) dans laquelle $R_1$ à $R_4$ représentent des radicaux alkyles linaires ou ramifiés, des radicaux cycloalkyles, aralkyles (par exemple benzyle) ou aryles monocycliques, ayant au

plus 16 atomes de carbone, pouvant le cas échéant être substitués par 1 à 3 radicaux alkyles ayant de 1 à 4 atomes de carbone ; deux des radicaux $R_1$ à $R_4$ pouvant éventuellement former ensemble un radical unique divalent — alkylène ou alcénylène — comportant 3 à 6 atomes de carbone (par exemple un radical tétraméthylène ou hexaméthylène) et le cas échéant 1 ou 2 doubles liaisons éthyléniques, ledit radical pouvant porter 1 à 3 substituants alkyles ayant de 1 à 4 atomes de carbone.

10. Procédé selon la revendication 8, caractérisé en ce que l'un quelconque des radicaux $R_1$ à $R_4$ est choisi parmi les radicaux alkyles linéaires ayant de 1 à 4 atomes de carbone.

11. Procédé selon la revendication 8, caractérisé en ce que le cation de l'iodure ionique est choisi parmi les cations phosphonium quaternaire de formule (I) dans laquelle l'un quelconque des radicaux $R_1$ à $R_4$ représente un radical alkyle linéaire ayant de 1 à 4 atomes de carbone, les trois autres radicaux étant identiques et choisis parmi les radicaux phényle, tolyle ou xylyle.

12. Procédé selon l'une quelconque des revendications précédentes, caractérisé en ce que la quantité d'iodure ionique chargée est telle que le rapport $I^-/Ru$ soit compris entre 1 et 100, et de préférence entre 2 et 50.

13. Procédé selon l'une quelconque des revendications précédentes, caractérisé en ce que la quantité totale de promoteurs iodés présente dans le milieu réactionnel est telle que le rapport atomique I/Ru soit compris entre 10 et 50.

14. Procédé selon l'une quelconque des revendications précédentes, caractérisé en ce qu'on charge en outre un cocatalyseur acalin ou alcalino-terreux.

15. Procédé selon la revendication 14, caractérisé en ce que le cocatalyseur est choisi parmi les acétates de lithium, de sodium, de potassium ou de magnésium.

16. Mode d'exécution du procédé défini par l'une quelconque des revendications 1 à 7, caractérisé en ce qu'on charge initialement de l'iodure de méthyle, le rapport molaire $CH_3$ I/Ru étant supérieur ou égal à 5 et un acétate de lithium, de sodium, de potassium ou de magnésium.

17. Procédé selon l'une quelconque des revendications précédentes, caractérisé en ce que la température est comprise entre 190 et 230 °C et la pression est comprise entre 150 et 350 bars.

18. Procédé selon l'une quelconque des revendications précédentes, caractérisé en ce que le rapport molaire $H_2/CO$ est compris entre 1/5 et 5/1 et, de préférence entre 1/1 et 3/1.

**Claims**

1. Process for the preparation of ethyl carboxylates by reacting a mixture containing carbon monoxide and hydrogen with the corresponding methyl carboxylates, in the liquid phase, at a temperature of between 175 and 250 °C, under a total pressure of between 50 and 600 bars, in the presence of ruthenium and an iodine-containing promoter, the atomic ratio I/Ru being greater than or equal to 5, characterised in that the reaction is carried out in the simultaneous presence of cobalt, the atomic ratio Co/Ru being less than or equal to 1, at least one alkyl iodide and at least one inorganic or organic ionic iodide in which the cation is chosen from the group comprising alkali metal cations, alkaline earth metal cations and quaternary ammonium or phosphonium cations.

2. Process according to Claim 1, characterised in that the methyl carboxylate has the formula $R—CO—OCH_3$, in which R represents a linear or branched alkyl radical having from 1 to 16 carbon atoms, a cycloalkyl radical having from 3 to 6 carbon atoms, a phenyl radical ($C_6H_5—$), a radical $C_6H_5—C_xH_{2x}—$ or a radical $C_xH_{2x+1}—C_6H_4—$, x being an integer between 1 and 6 inclusive.

3. Process according to Claim 2, characterised in that R represents an alkyl radical having at most 4 carbon atoms.

4. Process according to Claim 1, characterised in that the amount of ruthenium is between 0.5 and 100 mg atoms and preferably between 1 and 50 mg atoms per litre of reaction medium.

5. Process according to Claim 1 or 4, characterised in that the atomic ratio Co/Ru is between 0.01 and 1 and preferably between 0.02 and 0.50.

6. Process according to Claim 1, characterised in that the alkyl iodide used is methyl iodide.

7. Process according to any one of the preceding claims, characterised in that the cation of the ionic iodide is chosen from amongst alkali metal cations.

8. Process according to any one of Claims 1 to 6, characterised in that the cation of the ionic iodide is chosen from amongst the quaternary ammonium or phosphonium cations of the formulae I to III below :

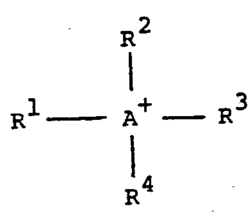

(I)

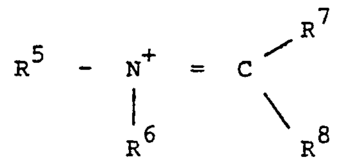 (II)

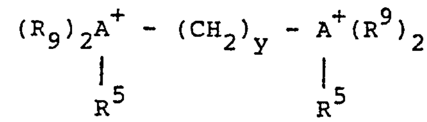 (III)

in which

A represents a nitrogen or phosphorus atom ;

$R_1$, $R_2$, $R_3$ and $R_4$, which can be identical or different, represent hydrogen or, preferably, organic radicals of which the free valency is carried by a carbon atom, it being possible, if appropriate, for any two of these various radicals together to form a single divalent radical ;

$R_5$, $R_6$, $R_7$ and $R_8$, which are identical or different, represent alkyl radicals having from 1 to 4 carbon atoms, it also being possible for one of the radicals $R_7$ or $R_8$ to represent hydrogen, and it being possible, if appropriate, for $R_7$ and $R_8$ together to form a single divalent alkylene radical containing from 3 to 6 carbon atoms, for example tetramethylene or hexamethylene ; $R_6$ and $R_7$ or $R_8$ can together form a single divalent alkylene or alkenylene radical containing 4 carbon atoms and, if appropriate, 1 or 2 ethylenic double bonds, the nitrogen atom in that case being included in a heterocyclic ring ; and

$R_9$, which can be identical to $R_5$, represents an alkyl radical having from 1 to 4 carbon atoms or a phenyl radical, and y is an integer between 1 and 10 inclusive and preferably between 1 and 6 inclusive.

9. Process according to Claim 8, characterised in that the cation of the ionic iodide is chosen from amongst the cations of the formula (I) in which $R_1$ to $R_4$ represent linear or branched alkyl radicals or monocyclic cycloalkyl, aralkyl (for example benzyl) or aryl radicals having at most 16 carbon atoms, which can be substituted, if appropriate, by 1 to 3 alkyl radicals having from 1 to 4 carbon atoms, it being possible, if appropriate, for two of the radicals $R_1$ to $R_4$ together to form a single divalent alkylene or alkenylene radical containing 3 to 6 carbon atoms (for example a tetramethylene or hexamethylene radical) and, if appropriate, 1 or 2 ethylenic double bonds, and it being possible for the said radical to carry 1 to 3 alkyl substituents having from 1 to 4 carbon atoms.

10. Process according to Claim 8, characterised in that any one of the radicals $R_1$ to $R_4$ is chosen from amongst linear alkyl radicals having from 1 to 4 carbon atoms.

11. Process according to Claim 8, characterised in that the cation of the ionic iodide is chosen from amongst the quaternary phosphonium cations of the formula (I) in which any one of the radicals $R_1$ to $R_4$ represents a linear alkyl radical having from 1 to 4 carbon atoms, the other three radicals being identical and chosen from amongst phenyl, tolyl or xylyl radicals.

12. Process according to any one of the preceding claims, characterised in that the amount of ionic iodide introduced is such that the ratio $I^-/Ru$ is between 1 and 100 and preferably between 2 and 50.

13. Process according to any one of the preceding claims, characterised in that the total amount of iodinecontaining promoters present in the reaction medium is such that the atomic ratio I/Ru is between 10 and 50.

14. Process according to any one of the preceding claims, characterised in that an alkali metal co-catalyst or alkaline earth metal co-catalyst is also introduced.

15. Process according to Claim 14, characterised in that the co-catalyst is chosen from amongst the acetates of lithium, sodium, potassium and magnesium.

16. Method of carrying out the process defined by any one of Claims 1 to 7, characterised in that methyl iodide is introduced initially, the molar ratio $CH_3I/Ru$ being greater than or equal to 5, together with lithium acetate, sodium acetate, potassium acetate or magnesium acetate.

17. Process according to any one of the preceding claims, characterised in that the temperature is between 190 and 230 °C and the pressure is between 150 and 350 bars.

18. Process according to any one of the preceding claims, characterised in that the molar ratio $H_2/CO$ is between 1/5 and 5/1 and preferably between 1/1 and 3/1.

**Ansprüche**

1. Verfahren zur Herstellung von Carbonsäure-ethylestern durch Umsetzen der entsprechenden Carbonsäure-methylester mit einem Gemisch, das Kohlenmonoxid und Wasserstoff enthält, in flüssiger Phase, bei einer Temperatur im Bereich von 175 bis 250 °C, unter einem Gesamtdruck im Bereich von 50 bis 600 bar und in Gegenwart von Ruthenium und einem iodhaltigen Promotor, wobei das Atomverhältnis $I/Ru \geqslant 5$ ist, dadurch gekennzeichnet, daß man gleichzeitig in Gegenwart von Cobalt arbeitet, wobei das

Atomverhältnis Co/Ru ≤ 1 ist, sowie in Gegenwart mindestens eines Alkyliodids und mindestens eines ionischen anorganischen oder organischen Iodids, dessen Kation ausgewählt wird aus der Gruppe, bestehend aus Kationen der Alkali- und Erdalkalimetalle sowie der Ammonium- oder quaternären Phosphoniumkationen.

2. Verfahren nach Anspruch 1, dadurch gekennzeichnet, daß der Carbonsäure-methylester der Formel $R—CO—OCH_3$ entspricht, in der R eine lineare oder verzweigte Alkylgruppe mit 1 bis 16 Kohlenstoffatomen, eine Cycloalkylgruppe mit 3 bis 6 Kohlenstoffatomen, die Phenylgruppe ($C_6H_5—$), eine Gruppe $C_6H_5—C_xH_{2x}—$ oder eine Gruppe $C_xH_{2x+1}—C_6H_4—$, bedeutet, wobei x eine ganze Zahl von 1 bis 6 ist.

3. Verfahren nach Anspruch 2, dadurch gekennzeichnet, daß R für eine Alkylgruppe mit höchstens 4 Kohlenstoffatomen steht.

4. Verfahren nach Anspruch 1, dadurch gekennzeichnet, daß der Anteil an Ruthenium je Liter Reaktionsmedium 0,5 bis 100 mgAtom, vorzugsweise 1 bis 50 mgAtom ausmacht.

5. Verfahren nach Anspruch 1 oder 4, dadurch gekennzeichnet, daß das Atomverhältnis Co/Ru 0,01 bis 1 und vorzugsweise 0,02 bis 0,50 beträgt.

6. Verfahren nach Anspruch 1, dadurch gekennzeichnet, daß als Alkyliodid Methyliodid eingesetzt wird.

7. Verfahren nach einem der vorangegangenen Ansprüche, dadurch gekennzeichnet, daß das Kation des ionischen Iodids ausgewählt wird unter den Kationen der Alkalimetalle.

8. Verfahren nach einem der Ansprüche 1 bis 6, dadurch gekennzeichnet, daß das Kation des ionischen Iodids das Ammoniumkation oder ein quaternäres Phosphoniumkation der folgenden Formeln I bis III

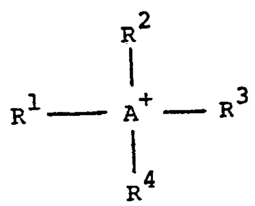 (I)

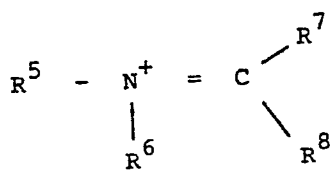 (II)

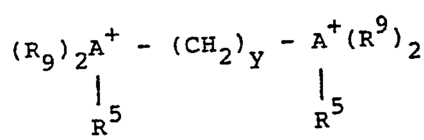 (III)

ist, in denen

A für ein Stickstoff- oder Phosphoratom steht,

$R^1$, $R^2$, $R^3$ und $R^4$ gleich oder verschieden sein können und jeweils für Wasserstoff oder vorzugsweise für organische Gruppen stehen, deren freie Wertigkeit von einem Kohlenstoffatom ausgeht, wobei zwei beliebige dieser verschiedenen Gruppen gegebenenfalls zusammen eine einzige zweiwertige Gruppe bilden,

$R^5$, $R^6$, $R^7$ und $R^8$ gleich oder verschieden sein können und für Alkylgruppen mit 1 bis 4 Kohlenstoffatomen stehen, eine der Gruppen $R^7$ oder $R^8$ außerdem Wasserstoff bedeuten kann, $R^7$ und $R^8$ gegebenenfalls zusammen eine einzige zweiwertige Alkylengruppe mit 3 bis 6 Kohlenstoffatomen, beispielsweise Tetramethylen oder Hexamethylen bilden, $R^6$ und $R^7$ oder $R^8$ zusammen eine einzige zweiwertige Alkylen- oder Alkenylengruppe mit 4 Kohlenstoffatomen und gegebenenfalls ein oder zwei ethylenischen Doppelbindungen bilden können, wobei das Stickstoffatom dann in einen Heterocyclus eingeschlossen ist,

$R^9$ identisch sein kann mit $R^5$ und für eine Alkylgruppe mit ein bis 4 Kohlenstoffatomen oder für die Phenylgruppe steht und y eine ganze Zahl von 1 bis 10, vorzugsweise von 1 bis 6 ist.

9. Verfahren nach Anspruch 8, dadurch gekennzeichnet, daß das Kation des ionischen Iodids ausgewählt wird aus den Kationen der Formel (I), in der $R^1$ bis $R^4$ lineare oder verzweigte Alkylgruppen, Cycloalkylgruppen, Aralkylgruppen (beispielsweise Benzyl) oder monocyclische Arylgruppen, mit höchstens 16 Kohlenstoffatomen und gegebenenfalls substituiert durch 1 bis 3 Alkylgruppen mit 1 bis 4 Kohlenstoffatomen, bedeuten, zwei der Gruppen $R^1$ bis $R^4$ gegebenenfalls zusammen eine einzige zweiwertige Gruppe bilden — Alkylen- oder Alkenylengruppe — die 3 bis 6 Kohlenstoffatome enthält

(beispielsweise die Tetramethylen- oder Hexamethylengruppe) und gegebenenfalls ein oder zwei ethylenische Doppelbindungen, wobei diese Gruppe mit 1 bis 3 Alkylgruppen, enthaltend 1 bis 4 Kohlenstoffatome, substituiert sein kann.

10. Verfahren nach Anspruch 8, dadurch gekennzeichnet, daß eine beliebige der Gruppen $R^1$ bis $R^4$ ausgewählt wird unter den linearen Alkylgruppen mit 1 bis 4 Kohlenstoffatomen.

11. Verfahren nach Anspruch 8, dadurch gekennzeichnet, daß das Kation des ionischen Iodids ausgewählt wird unter den quaternären Phosphoniumkationen der Formel (I), in der eine beliebige der Gruppen $R^1$ bis $R^4$ eine lineare Alkylgruppe mit 1 bis 4 Kohlenstoffatomen bedeutet und die drei anderen Gruppen identisch sind und jeweils für eine Phenyl-, Toluyl- oder Xylylgruppe stehen.

12. Verfahren nach einem der vorangehenden Ansprüche, dadurch gekennzeichnet, daß soviel ionisches Iodid aufgegeben wird, daß das Verhältnis $I^-/Ru$ 1 bis 100 und vorzugsweise 2 bis 50 beträgt.

13. Verfahren nach einem der vorangehenden Ansprüche, dadurch gekennzeichnet, daß die Gesamtmenge an im Reaktionsmedium vorhandenen iodhaltigen Promotoren einem Atomverhältnis I/Ru von 10 bis 50 entspricht.

14. Verfahren nach einem der vorangehenden Ansprüche, dadurch gekennzeichnet, daß man zusätzlich einen Alkali- oder Erdalkali-Cokatalysator aufgibt.

15. Verfahren nach Anspruch 14, dadurch gekennzeichnet, daß der Cokatalysator ausgewählt wird unter Lithiumacetat, Natriumacetat, Kaliumacetat oder Magnesiumacetat.

16. Ausführungsform des Verfahrens nach einem der Ansprüche 1 bis 7, dadurch gekennzeichnet, daß man Methyliodid entsprechend einem Molverhältnis $CH_3I/Ru \geqslant 5$ sowie ein Lithiumacetat, Natriumacetat, Kaliumacetat oder Magnesiumacetat vorlegt.

17. Verfahren nach einem der vorangehenden Ansprüche, dadurch gekennzeichnet, daß die Temperatur im Bereich von 190 bis 230 °C und der Druck im Bereich von 150 bis 350 bar liegt.

18. Verfahren nach einem der vorangehenden Ansprüche, dadurch gekennzeichnet, daß das Molverhältnis $H_2/CO$ 1 : 5 bis 5 : 1 und vorzugsweise 1 : 1 bis 3 : 1 beträgt.